(19) Europäisches Patentamt
European Patent Office
Office européen des brevets

(11) **EP 3 888 556 A1**

(12) # EUROPEAN PATENT APPLICATION

(43) Date of publication:
**06.10.2021 Bulletin 2021/40**

(21) Application number: **20167460.3**

(22) Date of filing: **01.04.2020**

(51) Int Cl.:
*A61B 8/00* *(2006.01)*        *G01S 15/89* *(2006.01)*
*G01S 7/52* *(2006.01)*        *G01S 7/539* *(2006.01)*
*G06K 9/32* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SCHNELLBÄCHER, Nikolas David**
**5656 AE Eindhoven (NL)**

• **WISSEL, Tobias**
**5656 AE Eindhoven (NL)**
• **NICKISCH, Hannes**
**5656 AE Eindhoven (NL)**
• **GRAß, Michael**
**5656 AE Eindhoven (NL)**

(74) Representative: **Philips Intellectual Property & Standards**
**High Tech Campus 5**
**5656 AE Eindhoven (NL)**

(54) **FOCUS OPTIMIZATION FOR PREDICTION IN MULTI-FREQUENCY ULTRASOUND IMAGING**

(57) An imaging system (IS), comprising an image acquisition unit (AQ) for acquisition of image data (I1) of an object (OB). The image acquisition is based on an imaging signal imitable by the unit (AQ) to interact with the object. The image acquisition unit (AQ) is adjustable to operate at different acquisition parameters that determine a property of the imaging signal. A predictor component (PC) predicts, based at least on the acquired image data (I1), one or more properties of the object. An acquisition parameter adjuster (PA) adjusts, based on the predicted object properties, the acquisition parameter at which the image acquisition unit (AQ) is to acquire follow-up image data (12).

**FIG. 1**

Printed by Jouve, 75001 PARIS (FR)

**Description**

FIELD OF THE INVENTION

The invention relates to an imaging system, to an imaging method, to a training method for a predictor component, to a computer program element, and to a computer readable medium.

BACKGROUND OF THE INVENTION

Intravascular ultrasound (IVUS) plays an increasing role for vascular imaging in coronary as well as peripheral vessels.

Applications include evaluation of plaque or calcium burden, stenosis level or verifying implant placement.

Current US transducer technologies include PMUTs or CMUTs. Either one can be designed for multi-frequency IVUS applications.

In such technologies, one or more vibrating elements ("active source"), configurable as a piezo-resistor or a capacitive membrane, transmit and receive different carrier frequencies. In many system, a discrete set or array of such transducer elements, each tunable to a certain frequency.

In IVUS applications, image quality (IQ) such as image resolution, noise level, but other properties such as the size of the field-of-view (FoV) are a function of the used transducer frequency. For example, low frequencies decrease image resolution but enlarge the FoV and result in low noise characteristics. With higher frequencies, the resolution increases whereas the FoV decreases. This renders lower frequencies more suitable to detect and delineate calcium arcs and lumen borders, while higher frequencies work better for the detection of for example TCFA (thin-cap fibroatheroma), a special type of plaque, since the fiber cap is only a few $\mu$m thick. High frequency settings might also be better suited to detect thrombi, because they provide an enlarged view on the vessel lumen and possibly improved discrimination from normal blood.

There is hence a tradeoff between imaging properties or IQ, such as image resolution, penetration depth and noise characteristics, in dependence on the used signal frequency, as different imaging tasks may call for different frequency regimes for best performance, such as classification or other.

SUMMARY OF THE INVENTION

There may be a need for improving imaging.

The object of the present invention is solved by the subject matter of the independent claims where further embodiments are incorporated in the dependent claims. It should be noted that the following described aspect of the invention equally applies to the imaging method, to the training method, to the computer program element, and to the computer readable medium.

According to a first aspect of the invention there is provided an imaging system, comprising:

an image acquisition unit for acquisition of image data of at least a part of an object, the said acquisition being based on an imaging signal imitable by the unit to interact with the object, the image acquisition unit adjustable to operate at different acquisition parameters that determine at least in part a property of the imaging signal;
a predictor component configured to predict, based at least on the acquired image data, one or more properties of the object; and
an acquisition parameter adjuster configured to adjust, based on the predicted one or more properties, the acquisition parameter at which the image acquisition unit is to acquire follow-up image data.

The system is preferably operable in real-time with prediction and/or adjustment done on the fly.

In embodiments of the medical field mainly envisaged herein, the object includes one or more human (internal) anatomies, or part thereof, such an organ or organ group. The acquisition is preferably configured to acquire imagery of internal anatomies.

The properties of the object include any one or more of: an identity/name or type, a finding or diagnosis, one or more measurements, and so on. Because certain object properties are of higher interest to certain imaging objectives/purposes or clinical tasks than to others, the object properties can be said to be linked more broadly to a type of clinical task/imaging purposes to be performed with respect of the object.

The prediction may include a classification, segmentation, delineation, or regression into measurement(s), or a combination of some or all of the foregoing.

The proposed system overcomes the above describe task-specificity and tradeoff of a single imaging parameter setting by automatically "focusing" the tunable imaging parameter to the best, or better, value for a given object property, and hence clinical task to be performed. For example, the system may help automatically "focus" the tunable IVUS frequency to the best value for a given segmentation.

Instead of acting on the image data directly, the system analyses the image data first to make predictions in relation to the object properties, and then adjusts the acquisition parameters accordingly based on the object property prediction to increase image quality on-line, in a feedback loop, and with better robustness. The system furnishes an autofocus capability where the acquisition unit are adjusted to improve an imaging objective as per the predicted object property.

The proposed system and method improves in particular (but not only) multi-frequency IVUS systems for intravascular imaging or other imaging purposes. The proposed system provides an automated frequency steering based on prediction performance and prediction uncertainty. This removes potential human bias from the acquisition and frequency selection. In addition, the system results in a better overall prediction performance and allows an optimized workflow for multi frequency IVUS imaging applications.

In embodiments, the acquisition parameter is adjusted automatically by the acquisition parameter adjuster so as to increase image quality.

In embodiments, the predictor component is to predict plural object properties associated with a respective uncertainty value, wherein the acquisition parameter adjuster is to adjust the acquisition parameter based on the uncertainty value or on a gradient thereof.

In embodiments, the acquisition parameter adjuster is to adjust the acquisition parameter so as to decrease the uncertainty value of the predicted one or more properties. Specifically, the predictor component may automatically provide an uncertainty estimation alongside with its prediction, to allow the said parameter adjuster to "steer" the acquisition parameter according to lowest / optimal prediction uncertainty.

In embodiments, the predictor component is to predict the one or more object properties based on a current acquisition parameter.

In more detail, in some such embodiments, the predictor component computes predictions and associated uncertainty measures for a pre-specified discrete set of acquisition parameters (eg, the US operation frequencies). The adjuster then selects the image or label associated with the lowest uncertainty measure (or with uncertainty measure less than as threshold), and stores the related frequency setting for follow-up image acquisitions. New predictions and their associated uncertainties are then made based on imagery acquired with the so stored imaging parameter. If the associated uncertainty is below a set threshold, the frequency settings is maintained. If not, a new scan of the frequency range is performed to find the new best minimal uncertainty and the associated imaging parameter setting which is then again stored for the follow-up acquisition, and so on.

In another embodiment, the predictor component initializes the system by making an initial prediction based on a reference parameter, such as mid-range operation frequency or other reference frequency. The parameter adjuster may be configured to compute the gradient of the label uncertainty with respect to the frequency input, and by moving along the gradient to adjust the frequency so as to decrease the prediction uncertainty.

In other embodiments, the predictor component first predicts the presence of a particular label, the parameter adjuster adjusts the imaging parameter (eg, the transducer frequency) to a better, or even optimal one for those labels as known from prior knowledge. The prior knowledge may be represented in a pre-set data structure such as a look-up table other where prediction labels are stored in association with respective best frequencies. The prior knowledge data structure can be dynamically built up by the system itself in a preparatory or exploratory phase where scan is performed by acquiring a series of images whilst changing the imaging parameter in a range.

In embodiments, the uncertainty value is provided by a user via a user interface. Specifically, the parameter adjustment is based not only on the predictor component output, but is further based on a human expert interaction signal ("human in the loop"), to iteratively steer the acquisition process based on human expert knowledge.

In embodiments, the predictor component includes a pre-trained machine learning model.

In embodiments, the pre-trained machine learning model includes a neural network. The neural network may include one or more hidden layers, in particular one or more convolutional layers.

In embodiments, the image acquisition unit includes a multi-frequency ultrasound imaging device, wherein the imaging signal is an ultrasound signal. However, other imaging modalities, preferably those based on non-ionizing imaging signals are also envisaged herein, such as optical coherence tomography or others still.

In embodiments, the acquisition parameter includes a frequency of the ultrasound signal. Other imaging parameters of an US imaging system that are determinative of the US-signal are also included herein, in addition or instead of the US frequency. When other imaging systems other US are used, the imaging parameter relates to other quantities that are adjustable and determinative of properties of the imaging signal, and hence of IQ and/or imageable properties of interest of the respective object.

In embodiments, the ultrasound imaging device is an intravascular ultrasound, IVUS, imaging device

According to another aspect of the invention there is provided a training system configured to train the predictor component.

According to another aspect of the invention there is provided an imaging method, comprising the steps of:

acquiring image data of at least a part of an object, the said acquiring being based on an imaging signal emittable by an image acquisition unit to interact with the object, the image acquisition unit adjustable to operate at different acquisition parameters that determine at least in part a property of the imaging signal;
predicting, based at least on the acquired image data, one or more properties of the object; and
adjusting, based on the predicted one or more properties, the acquisition parameter at which the image acquisition unit is to acquire follow-up image data.

In an optional step of the method, the predicted labels and/or findings are visually localized in the current image by graphical elements. The graphical elements may indicate tissue boundaries etc. The graphical elements may be displayed on a display device concurrently with the current image. For example, the graphical element(s) may be overlaid on the currently displayed image.

According to another aspect of the invention there is provided a training method configured to train the predictor component.

In another aspect there is provided a computer program element, which, when being executed by at least one processing unit, is adapted to cause the processing unit to perform the method as per any one of the above mentioned embodiments.

In another aspect still, there is provided a computer readable medium having stored thereon the program element.

*"user"* relates to a person, such as medical personnel or other, operating the imaging system or overseeing the imaging procedure. In other words, the user is in general not the patient.

*"object"* is used herein in the general sense to include animate "objects" such as a human or animal patient, or anatomic parts thereof but may also include inanimate objects such as an item of baggage in security checks or a product in non-destructive testing. However, the proposed system will be discussed herein with main reference to the medical field, so we will be referring to the "object" as "the patient".

*"lD, "2D",* or "3D" or *"nD",* refers to dimension *n, n=1,2,3,* .., so *"lD"* means "one-dimension(al)", "2D" means "two-dimension(al)", and so on.

*"US"* is shorthand for ultrasound, the imaging modality mainly (but not exclusively) envisaged herein.

*"GPU"* relates to processors with architectures that support parallel processing such as graphics processing units or others.

In general, the *"machine learning component"* is a computerized arrangement that implements a machine learning ("ML") algorithm that is configured to perform a task. In an ML algorithm, task performance improves measurably after having provided the arrangement with more training data. The task's performance may be measured by objective tests when feeding the system with test data. The task's performance may be defined in terms of a certain error rate to be achieved for the given test data. See for example, T. M. Mitchell, "Machine Learning", page 2, section 1.1, McGraw-Hill, 1997.

## BRIEF DESCRIPTION OF THE DRAWINGS

Exemplary embodiments of the invention will now be described with reference to the following drawings, which, unless stated otherwise, are not to scale, wherein:

Figure 1 shows a schematic block diagram of an imaging system;
Figure 2 shows a schematic block diagram of a processing components of the imaging system;
Figure 3 shows a schematic block diagram of a processing component according to one embodiment;
Figure 4 shows a schematic block diagram of a processing component according to a second embodiment;
Figure 5 shows a schematic block diagram of a processing component according to a third embodiment;
Figure 6 shows a flow chart of an imaging method;
Figure 7A shows a schematic block diagram of a machine learning model;
Figure 7B shows a schematic block diagram of a training system for training a machine learning model; and
Figure 8 shows a flow chart of a method of training a machine learning model.

## DETAILED DESCRIPTION OF EMBODIMENTS

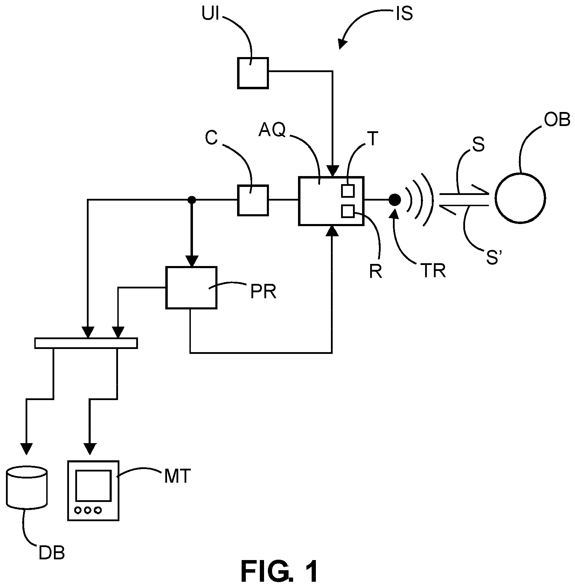

With reference to Figure 1 there is shown a schematic block diagram of an imaging system IS.

The imaging system IS may be configured for medical imaging, but other application outside the medical field are not excluded herein.

Broadly, the imaging system IS includes an image acquisition unit AQ. The image acquisition unit AQ is operable to emit an imaging or "interrogating" signal S. The imaging signal S is configured to "interrogate" an object OB or certain of its one or more properties of interest. Specifically, the imaging signal S is to interact with an object of interest OB such as a patient or an anatomic part thereof. The imaging signal S interacts with patient tissue OB. The interrogation signal S is modified as a result of this interaction, and a modified signal S' is then received at the image acquisition unit AQ.

The incoming, modified signal S' as received at the acquisition unit AQ is digitized by a converter C into numerical data. The numerical data can be further processed or converted into image date. The image data can be stored on a database DB, visualized on a display unit MT, or can be otherwise processed.

The acquisition unit AQ is preferably operable to emit a non-ionizing imaging signal S. The image acquisition unit AQ and the image processor PR may be integrated into a single housing (not shown), but this is not necessarily the case in all embodiments. The processing unit PR may be situated outside the housing, away and remote from the acquisition unit AQ, and the two may communicate through a wireless or a wired communication arrangement. In addition, the display device or monitor MT on which the acquired imagery may be displayed, may be integrated into the housing, together with the acquisition unit and/or the processing unit PR. But again, such an integration of components is not necessarily the case in all embodiments. Specifically, the monitor MT may instead be situated outside the housing, and the acquired imagery is forwarded to the monitor MT for display through a wired or wireless connection. The acquisition unit AQ may be operable to acquire a stream of images which can then be displayed as a video feed on the monitor MT.

In embodiments mainly envisaged herein the image system is arranged as an ultrasound imaging system. However, other non-ionizing-signal-based imaging systems IS are also envisaged, such as optical coherence tomography OCT, in particular, but not only, intra-vascular IVOCT. The non-ionizing imaging system preferably envisaged herein are capable of acquiring real time imagery.

A particular field of application envisaged herein in some embodiments is intravascular ultrasound, IVUS, but this does not to exclude other ultrasound imaging systems such as externally applied systems. The imaging system may be cart-based, portable, hand-held or, as said, endoscopic and/or intravascular.

In ultrasound systems, but also in other imaging systems, the acquisition unit AQ includes a transducer TR. It is through the transducer TR that the US signal S is emitted and received as the modified signal S'. Transducer T us coupled to transmitter T and receiver R circuitry of the acquisition unit AQ. The transmit and receive circuitry T,R may be integrated into a single sub-unit, a transceiver circuitry, or may be arranged separately. The transmit and receive circuitry operate as switches in turns. The transmit circuitry T switches the transducer TR in a mode to emit the US signal S. The receive circuitry R switches the transducer in a mode to receive the modified (eg, reflected) signal S'. Whilst transducer TR can act to receive and transmit signals, this is not a necessary requirement herein for all embodiments. Alternatively, the outgoing signal S may be generated by one component, whilst the incoming signal is received by another (detector) component. This in particular the case for other imaging modalities, such as OCT or other still, but, although less preferred, may also be practiced in US imaging.

In US imaging, the transducer TR includes preferably plural active sources or transducer elements (not shown) that are each individually addressable to cause respective ultrasonic pressure waves at specific frequencies. Plural such sources may be arranged in a line or in a 2D array. The array or linear arrangement may be curved. Phased arrays are also envisaged.

Imaging characteristics such as image quality, in particular image resolution, noise level, size of field-of-view (FoV), etc. are a function of certain imaging acquisition parameters (referred to herein briefly as acquisition parameters) that are set automatically or by the user.

In ultrasound imaging as mainly envisaged herein, one such acquisition parameter is the ultrasound frequency. The ultrasound frequency describes the frequency with which the transducer element(s) is/are vibrating to cause the pressure waves that are coupled into the patient as imaging signals S to image the internals of the patient. The pressure waves at the given frequencies pass through patient tissue and are reflected off boundaries of different tissue types. A fraction of the incoming ultrasound wave S is absorbed whilst another fraction is scattered off or reflected S' and is then detected at the transducer of the acquisition unit AQ. Based on a time difference between emission of outgoing sonic wave S and reception of the back-reflected sonic wave S', a distance can be computed for different positions to so build up the image.

Whilst the transducer TR is mainly envisaged as an array comprised of a plural active sources, this is not necessarily the case in all embodiments and "mono"-source transducer with a single transducer element are also envisaged. Preferably however, a multi-source transducer is envisaged. The transducer elements may be arranged in PMUT or CEMUT technologies, or other.

Preferably, the imaging system is capable of multi-frequency operation. More particularly, the operating ultrasound frequency as one of the acquisition parameters can be changed either by the user or, preferably automatically and dynamically, as will be described more fully below. The ultrasound (US) frequency range envisaged herein depends on the application and equipment but will typical lie in the MHz-range, such as 1-40 MHz. For example, a typical US range for IVUS is about 10-40 MHz.

In embodiments, the acquisition parameter, such as the US frequency, can be dynamically changed in a feedback loop, in dependence on the acquired US imagery, to be explained in more fully below. Because the main focus herein is on ultrasound imaging the acquisition parameter may also be simply referred to herein as "the frequency", with the understanding however that all that has been said above, and all that will be explored below, is of equal application to imaging parameters other than US frequency.

The US frequency may describe a global frequency that describes the frequency of a combined ultrasound wave that is created by interference of individual "wavelets" generated by the individual transducer elements or sources. In addition or instead, the "frequency" is a matrix or vector quantity of local frequencies that describes, respectively, individual frequencies of the local waves, or wavelets, generated by the individual transducer elements in a phased, linear or curved array. In the notation $f=(f_i)$, $i$ are the transducer element positions $i$ in the array of the transducer elements TR. The transducer element position $i$ is associated with a respective pixel positon in the image. As the array may be ID or 2D, the space index $i$ is either ID (a scalar) or 2D ($i=i_1,i_2$)). In others words, frequency is a space dependent variable, depending on source and hence pixel position. In the following, whenever reference is made to "$f$" this notion is understood to include the case where $f$ is a vector or matrix.

As explained earlier, the image characteristics are a function of the frequency $f$ and there are usually trade-offs between different imaging tasks and objectives as observed earlier. The imaging system IS as proposed herein is configured to dynamically adapt. preferably in real time, in between individual image acquisitions of in imaging stream, the frequency for a given image task or objective as deduced by the ogic of the processing unit PR, based on the "semantics" of what is being imaged at a given instant. This semantics based processing makes the frequency adaptation more robust, as compared to approaches of adaption based on noisy raw image information as such, without such a "semantic layer". This semantic aspect will be explored more fully below.

Preferably, the ultrasound imaging system IS operates in a dynamic feed-back loop where a current image is analyzed by the processing unit PR and, based on this analysis, the frequency is adapted accordingly during acquisition of the stream of images. In particular, the frequency is adapted based on a given image in the stream, and the adapted frequency may then be used for the follow-up image and so on. If the analysis by the processing unit PR so reveals, the frequency may be kept constant as long as certain imaging objectives are met which is supervised by the processing unit PR. As will be explained more fully, the processing unit may be based particularly on a machine learning algorithm. In other words, the processing unit PR may incorporate a pre-trained machine learning model such as an artificial neural network or other that has been previously trained on training data. In addition to analyzing merely a current image, the processor may in addition base its analysis on the current frequency used for the current image to adjust the follow-up frequency for the next image. Whilst the imaging system as described is mainly envisaged to operate in a dynamic feed-back loop over the image stream, a user interface UI may still be provided to allow the human operator to "remain in the loop". The user can thus provide feedback-input, such as rating, on the image quality for instance, and this input is then taken into account by the processing unit PR to compute the new frequency which is then used to control the transducer to acquire the next follow-up image.

In general, the processing by the image processer PR proceeds for each image transducer element $i$ (scalar or 2D) separately to compute for each transducer element $i$ the associated frequency at which the respective transducer element $i$ is to be driven.

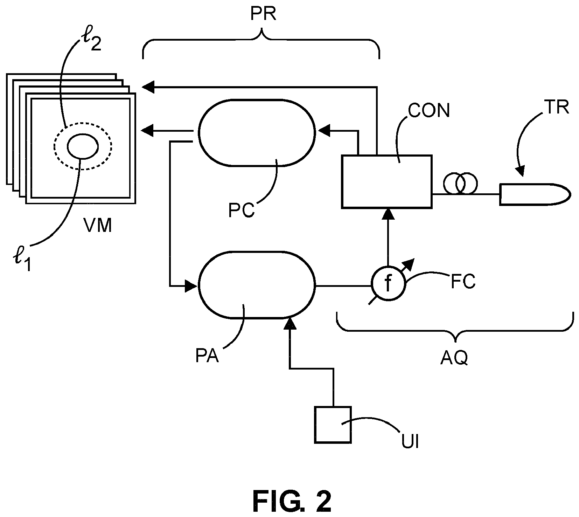

Reference is now made to the block diagram in Figure 2, which shows more details of the processing unit PR. For clarity, and to better explain interaction, parts of the acquisition unit AQ are also shown in Figure 2. In particular, as shown on the right of Figure 2, the acquisition module AQ includes the transducer TR. The frequency or frequencies of the transducer is controlled by a controller CON communicatively coupled to the transducer TR. The ultrasound controller CON energizes the transducer accordingly to generate the ultrasound signal at the prescribed frequency. The frequency is prescribed by a frequency controller FC and this is adjustable based on output received from the processing unit PR which is shown in the center potion of Figure 2.

In operation, images are acquired using a tunable transducer frequency via the acquisition module AQ. The acquired imagery is then passed on to prediction module PC. The prediction module PC may include a suitable trained machine learning ("ML") component, such as a pre-trained artificial neural network (NN). Other machine learning models or methodology is not excluded herein. The prediction module PC analyses the acquired imagery and generates predictions or labels $\ell_1$, $\ell_2$ which may be visualized by an optional visualization module VM together with the acquired imagery on the display unit MT. Graphical overlays may be used to visualize $\ell_1$, $\ell_2$ as indicated schematically in Figure 2 by circles in dashed and solid circles. The labels can be of all kinds as they can for example be generated from classification, segmentation or tracking algorithms based on the acquired imagery.

A feed-back parameter adjuster module PA receives and analyzes the imagery and/or the labels and may then propose a new frequency f' which is then passed on to the frequency controller. The frequency controller FC re-adjust the imaging frequency in real time accordingly to drive the transducer elements to produce US waves at the said new frequency f' to acquire the next image(s), and so on. Preferably, the operation of the feed-back module PA is based on the predicted labels, for instance segmentations of image structures in the received current image. In embodiments, the predictor PC attaches uncertainty values (or score or measures) to the predicted labels to quantify the prediction quality of the predicted labels. Preferably, the feed-back module PA uses the uncertainty scores produced by the prediction module PC to optimize the transducer frequency $f$ for better and more certain predictions. The feed-back module PA preferably adjusts the new frequency so as to reduce or minimize uncertainty values.

The prediction operation includes in particular computing of the labels $\ell_1$, $\ell_2$. The predictions may comprise a global label(s) for the whole image that represents for instance the overall anatomy. In extreme cases a single label for the whole images is predicted. Alternatively, or in addition, the prediction may include a plurality of labels $\ell_1$, $\ell_2$ for some or each image, where the labels relate to certain pixel/voxel sub-sets of the image. Single elements sub-sets are also envisaged. In other words, a labeling (such as a classification), with granularity down to individual pixels or voxel level is envisaged so that each pixel receives its own label. In coarser graining, the labeling may refer to sub-sets of more than one pixel/voxel. The prediction may hence be coarser or finer grained, and the granularity may be user adjustable or is pre-set. There may exists a plurality of pre-sets for various predictions tasks envisaged in this context.

Operation of the proposed processor PR is based on at least two functional relationships, concatenated with each other S ° M: First there is a predictor function:

$$P:\ \mathrm{I} \text{->} \ell \qquad\qquad (1)$$

preferably implementable as a machine learning algorithm, that maps imagery to labels. The labels in label space represents the semantics referred to above. The label indicates what each pixel, region, or whole image means in a clinical sense, which anatomy or part thereof is represented, what finding is indicated, etc, and similar for applications in other fields where different semantics may apply. The predictor Function P is implemented by predictor component PC.

Second, there is a selector function:

$$S:\ \ell \text{->} f \qquad\qquad (2)$$

implemented by the parameter adjuster PA. The selector maps labels onto the associated imaging parameter, in particular the US operating frequency $f$ mainly envisaged herein.

As the acquisition unit acquires a stream of imagery ("frames") in time and because each frequency is associated with a respective transducer source and hence pixel position, the selector function is in embodiments a space and time function S: $(\ell,i,t)$ -> $(f,i,t)$, with $i$ indicating, as introduced above, pixel/transducer element positon, and $t$ indicative of acquisition time.

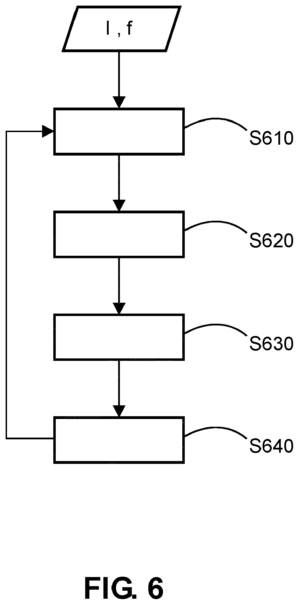

In embodiments, the labels $\ell$ as supplied by the predictor PC are associated herein with respective predictor uncertainties, denoted herein as "$\sigma$", and explained in more detail below at Figure 6. This uncertainty may be written as $\sigma_\ell$, the uncertainty associated with the respective label. In such embodiments, the selector function S may be configured as a function of uncertainty versus imaging parameter:

$$S:\ (\ell,i,t) \text{->} (\sigma,i,t) \text{->} (f,i,t) \qquad\qquad (3)$$

The selector function S may represent prior knowledge, and may be implemented as a look-up-data structure, for instance a look-up table (LUT), as interpolation in a LUT, as an analytic expression, or may be learned from a second machine learning model. The uncertainty values may be produced by the machine learning or other algorithm implemented by the predictor component PC when predicting the labels. Alternatively, the uncertainty values are produced by post-processing of the output produced by the predictor component. The notation "$\ell$" will be used herein to refer top output of the predictor PC and is to be construed broadly as any identifier of any of the above mentioned types of predictions envisaged herein.

The selector function S is in instances implicitly known, as the predictor function M may be understood as function of the imaging parameter. This is because the input image is itself a function of the imaging parameter, eg US frequency.

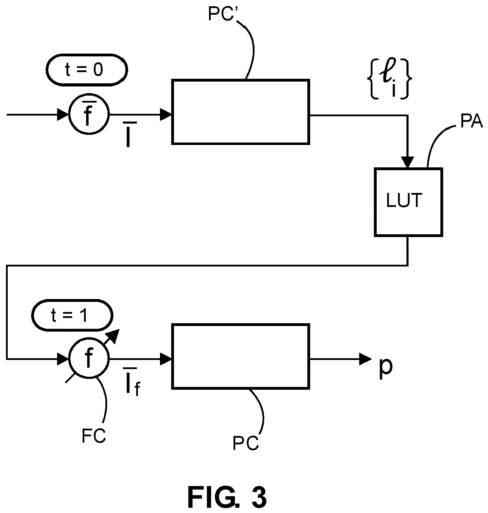

In embodiments the system PR may be configured to dynamically built up the LUT during acquisition, by storing all labels and/or uncertainties of some, preferably all, previously used frequencies. This LUT then represents prior knowledge about the best-frequency setting for a given label or detection task. Other implementations are also envisaged and will be discussed more fully below at Figures 3-5.

Preferably, the selector function S is smooth over $\sigma$, in particular is differentiable, and may also be smooth in space and/or time. The collection of all possible uncertainties values may be referred to herein as the "uncertainty space".

Explaining the operation of the imaging system IS in more detail, it is proposed to implement a predictive algorithm in particular for improved multi-frequency IVUS operation and label prediction. Preferably, the algorithm is based on a differentiable selector function S for improved multi-frequency IVUS operation and prediction. Broadly, the system takes acquired IVUS images (or image stacks) and optionally the current US frequency as inputs and returns predicted labels for the whole image (e.g. image classification) or on the single-pixel level (e.g. segmentation) as outputs. The label prediction is further complemented by providing a predictive uncertainty, following, e.g., a Bayesian uncertainty approach or other. By optimizing for low uncertainty predictions, the system solves the task of selecting an improved (e.g. the best) frequency setting per current image and/or prediction task, and dynamically tracking and/or automatically adapting the optimal operation frequency over time/space. Tracking allows dynamically building up the above mentioned prior knowledge in form of a LUT or other memory structure. It is assumed herein that the optimal frequency is a smooth function over space and time, which seems valid in most applications due to expected spatial correlations of images proximal in space and/or time.

Referring now to Figures 3-5, these show respective block diagrams of different embodiments and aspects of the imaging system's logic, in particular the inter-play between the prediction module PC and feed-back parameter adjuster PA.

In more detail, Figure 3 shows an embodiment that uses a "low-weight" pre-prediction model to detect global labels for an initial image(s). Based on prior knowledge, implemented for example in a LUT or similar data structure, the best frequency for some or each label is identified. New images may then be acquired, using the so determined frequency setting f. The prediction module PC may then in turn, thanks to a better IQ, predict improved (now possibly more detailed and better localized) predictions for each image.

In more detail, a mid-range frequency $\overline{f}$ may be used to generate the initial IVUS image $\overline{I}$ in an initial phase ($t=0$). From this input, a pre-prediction module PC' provides a set of predictions $\ell_i$ which are stored together with their optimal frequencies in a look up table (LUT) to gradually build up prior clinical knowledge. This information is then exploited in a second phase ($t=1$) to tune the frequency, leading to an updated image $I_f$ with enhanced predictions by operation of predictor PC. Alternatively, the LUT, where the labels are stored versus best frequencies, is pre-defined by a human expert.

Whilst Figure 3 shows the predictor PC separate from pre-predictor PC' as two components, and whilst this is indeed so envisaged in embodiments, such as separation into two components is not a requirement and pre-predictor PC' is merely a representation of predictor PC operating in preparatory phase in a different mode to build up the LUT. In alternative embodiments, there is neither such a pre-prediction module PC' nor such a mode, and the LUT is assembled in other ways. The pre-predictor PC', or the pre-predictor mode, is low-weight in terms of expected computing power as the demands are lower compared to the predictor or predictor mode PC. For instance, in this embodiment the pre-predictor PC' is merely tasked with building up the LUT as opposed to the higher computational demands on the predictor PC in predicting the imaging parameter $f$.

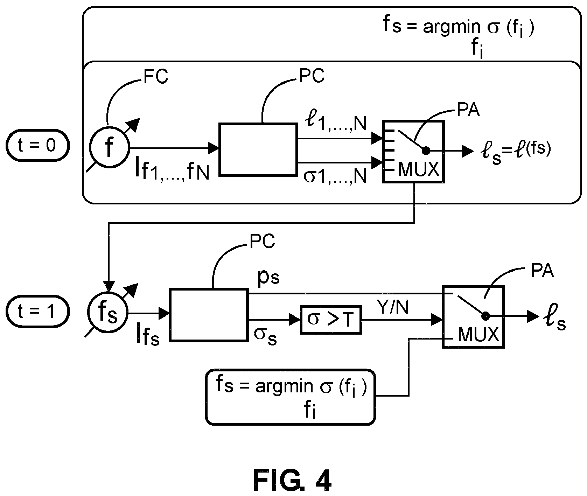

Referring now to Figure 4 this shows a different embodiment where the predictions for the frequencies $f$ are computed based on a pre-specified discrete set of operation frequencies. Then the image is selected S, with the lowest uncertainty measure. The frequency associated with this uncertainty is then used to acquire the next images. The transducer frequency is preferably only updated if the prediction uncertainty $\sigma$ for this frequency exceeds a pre-defined threshold value. In this case, some or all frequencies are revisited as described for the initial step, and the frequency may be adjusted as before. The system PR proceeds over some or all follow-up images in this iterative manner.

In more detail, in an initial iteration ($t = 0$), multiple images $I_{fi}$ are acquired in an exploratory phase by scanning over a range of available frequencies $f_i$ using initial or test images. For all or some of these test images (now associated with frequencies $f_i$), the prediction module PC outputs the predictions $\ell_i$ and preferably their associated uncertainties $\sigma_i$. The parameter adjuster PA, implementable in embodiments as a multiplexing element (MUX), selects S the label $\ell = p_s$ with lowest uncertainty or an uncertainty below a threshold, and stores the associated frequency setting $f_s$ for the next iteration ($t = 1$). A new image $I_{fs}$ may then be acquired with $f_s$, and the new image is then passed on to the prediction module PC. If the corresponding prediction uncertainty is below a threshold $T$, the prediction $\ell_i$ is kept. Otherwise, a new scan over the frequency range is performed to find the new best minimal uncertainty setting and this is then stored for the following iteration.

Whilst the embodiments in Figures 3,4 may require operation in respective preparatory phases, this can be implemented with very little delay in terms of user experience by using fast processors as such as GPUs or others.

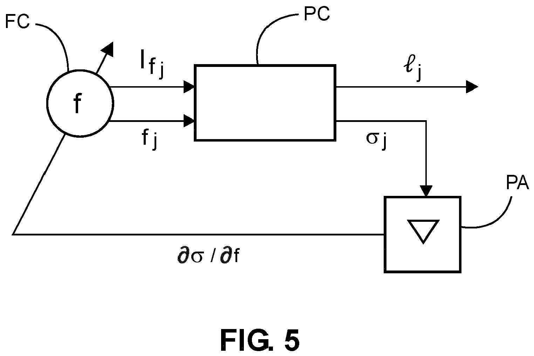

Referring now to Figure 5, this shows an embodiment where an initial prediction label, associated with an uncertainty, is predicted, by using an image acquired at an initial mid-range US frequency.

A gradient (indicated as "nabla" $\nabla$ in Figure 5) is then computed (by the adjuster PA or by another computational entity) for selector function S with respect to the frequency. As mentioned above, function S is implicitly known because the image is a function of the imaging parameter, in this case frequency $f$. The frequency is then adjusted by moving at a pre-defined or adaptively adjusted step (width) along the gradient direction in uncertainty space to a point of lower prediction uncertainty. This new frequency may then be used to acquire a new image, with better prediction performance.

For the following images, this gradient based *f*-adjustment is repeated. In embodiments, the previously found location along in uncertainty space (optimal frequency for the previous slice) may then be used as the initial starting point for the current iteration. The previously found location represents the optimal frequency for the previous image.

This gradient based method can be applied for continuously tunable transducers, preferably after clipping to appropriate frequency boundary conditions. The gradient-based scheme may also be applied to discretely tunable transducers. For discretely tunable transducers, the improved frequency is then selected from the discrete set of frequencies as the frequency closest to the location in uncertainty space as found in the above described gradient step.

In one embodiment of Figure 5, both, the current image $I_{fj}$ and the current frequency setting $f_j$ are fed into the prediction module PC and used to compute the gradient of the selector function S with respect to frequency. The error is fed back to the frequency adjuster PA. Adjuster PA then adjusts the current frequency based in the gradient. The adjuster then instructs frequency controller FC to drive the transducer at the adjusted frequency, thus reducing prediction uncertainty.

Using the current image along with the current frequency may also be used in the embodiments of Figures 3, 4.

In an alternative to the embodiments in Figures 3-5, or in addition, user input on the predictions as provided by the prediction module can be provided through a user interface UI. In other words, as an alternative to relying on the prediction uncertainty measure for optimizing the frequency, one can rely on feedback of a human operator in the loop. By interactively providing labels for certain anatomies, one can use the predicted label accuracy as an optimization criterion. In one embodiment, merely providing qualitative, binary feedback such as *"separation/segmentation or (overall) image quality of structure got better/worse / or current frame got better/worse"* can help to optimize for the frequency.

The above described embodiments in Figures 3-5, including the user feedback assisted option, provide a scheme that will dynamically shift the optimal operation frequency of the US-imaging system, and hence implements a frequency "auto-focus" for optimal prediction performance.

It will be understood that the predictor component PC and the parameter adjuster PA are arranged as two distinct functional components as shown in Figures 2-5. Alternatively, the two functionalities are merged and/or integrated into a single functional component.

Reference is now made to Figure 6 that shows a flow chart of an imaging method that may underlie operation of the above described embodiments of the imaging system IS. However, it will be understood that the following described steps of the imaging method are not necessarily tied to the architecture given in Figures 1-5, and may be understood as a teaching in their own right.

At step S610 an image at first instant $t=0$ is acquired at a first acquisition parameter *f* by an image acquisition unit of an imaging system. Imaging is based on the acquisition unit emitting an imaging or imaging signal that interacts with the object to be imaged and is then detected after interaction at the acquisition unit, and converted into imagery.

The acquisition parameter *f* is adjustable and determines certain characteristics of the imaging signal, and hence is determinative of image properties such as IQ, FoV, etc.

In embodiments, the image acquisition unit is one of an ultrasound imaging system and the imaging parameter is the US frequency of this multi-frequency ultrasound system. Other imaging modalities are not excluded herein, so long as the imaging signal is preferably non-ionizing.

In an ultrasound system the imaging or imaging signal is an ultrasound wave. In embodiments a transducer of the image acquisition unit includes multiple active sources, each addressable to emit its own sound wave so as to image different portions of the current field of view in the image acquisition.

The acquisition parameter includes the frequencies of the respective sound waves to be emitted by each source. The acquisition parameter is hence not necessarily a single parameter, but is a matrix or a vector that describes the respective ultrasound frequency of each of the active sources of the transducer. Not excluded herein, although less preferred, are transducers with a mono-source, in which case the frequency parameter is indeed a single scalar value describing the frequency of the pressure sound wave causable by said single source. The frequency as the acquisition parameter will be referred to herein as "*f*", no matter whether this is a scalar, vector or matrix quantity.

The image so acquired at the current frequency, is one of an object such as an internal anatomy or parts thereof of in a human or animal patient. For instance, in IVUS the transducer is attached to an endoscopic or catheter which is introducible into a blood vessel of the patient. Cardiac vessels may be imaged this way. In IVUS embodiments, the imaged object is then a part of the blood vessel, such as a cardiac vessel or other. The image provided by the IVUS image acquisition unit allows the user to obtain image-based information pertaining to the internal structure of the vessel wall for example. One exemplary imaging objective or task may be to ascertain and to distinguish between wall tissue of the vessel and layers of deposits on said wall, such as plaque, calcium, etc. This information may be used to assess severity of a stenosis where the vessel lumen is constricted due to the deposits. However, the following and the above is not confined to internally applicable ultrasound but is also applicable to ultrasound applied from the outside of the patient. Such external application may include coupling a lead portion of the transducer TR through a gel or other substance to the patient's body so as to admit the ultrasound wave inside the patient's body, such as is done for example during pregnancy examinations. But again, as mentioned above, imaging modalities other than ultrasound such as OCT or others are also envisaged. The imaging parameter as used herein is then construed accordingly to indicate other relevant image settings, such as the frequency of the light (typically near IR light) used in OCT, etc.

At step S620, based on the image acquired at the first parameter, one or more properties of one or multiple objects in the current field of view are predicted. The said properties will depend on the imaging task at hand but will include, for instance, identification of the one or more objects found in the current field of view and/or certain findings such stenosis "yes or no?", etc. as deducible from the predicted object properties.

As envisaged herein, the result of a prediction is a labeling or a segmentation/delineation of recognized image structures. The labels are encodings of the said identity or type of the object found in the image and/or the labels code for findings, etc. task envisaged, etc. For instance, as shown above in Figure 2, the labels may distinguish the deposition $\ell 2$ from the actual wall $\ell 1$ of the vessel. Respective segmentations may be formed based on the labels.

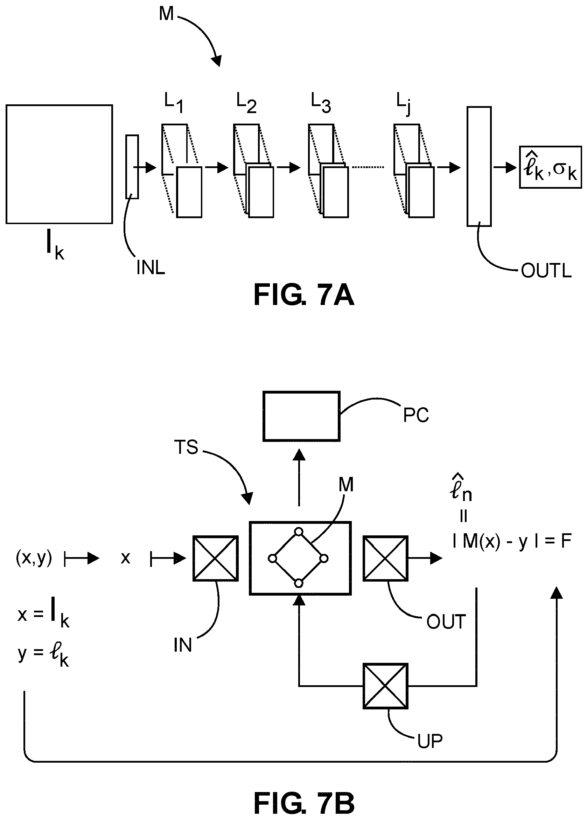

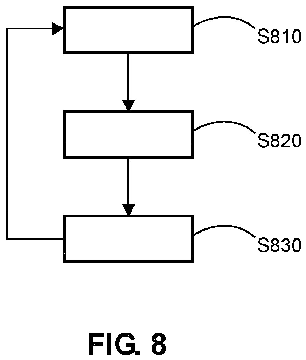

The prediction operation S620 is preferably implemented as a machine learning algorithm. In embodiments, a pre-trained artificial neural-network is used as has been mentioned above. Further particulars of a machine learning algorithm as envisaged herein will be explained in more detail below at Figures 7 and 8.

In an optional step, a visual indication such a color or otherwise coded overlay graphic is displayed overlaid on the current image on a display device.

At step S630 based on the predicted one or more labels, the current imaging parameter such as the frequency $f$ is adjusted, preferably for each source i, to obtain for some or each source $i$, a respective frequency $f_i$.

The newly adjusted imaging parameter $f$ may then be used in step S640 to acquire a follow-up image which is then again received at step S610 and the method proceeds with the new image as described above in a new loop, and so on.

The method can thus be seen to implement a dynamic feed-back loop that is based on an ongoing adaption of the image acquisition parameter at the chosen frame rate.

The adjustments at step S630 furnishes an "auto-focus" ability that dynamically updates the imaging parameter based on the properties of the one or more objects as predicted at prediction step S620. In other words, as the user is changing a direction of the current field of view, the parameters are co-adapted in response to the FoV change, in quasi-real time, as new properties (of possibly new objects) are predicted, which may hence call for the current acquisition parameter $f$ to be updated when further imaging for the now newly predicted object properties.

The adjustment at step S630 of the imaging parameters is preferably automatic and is done so as to increase an image quality such as resolution and/or to lower noise level. In addition or instead, the image parameter is to increase the FoV, depending on the image task at hand. In one embodiment the imaging task itself is deduced by the predictor component from the predicted labels, and the image acquisition parameter is then adapted accordingly. In other words, a smart imaging device can be provided by the proposed method where the system knows from the predicted properties of the image, what task the user wishes to accomplish and the image acquisition parameters are adjusted accordingly so as to be optimized to the deduced task.

Some machine learning algorithms as may be used in step S620 furnish one or more uncertainty values that are associated with the respective labels. The uncertainty values measure the amount of uncertainty associated with the prediction of the respective label. Machine learning methods with Bayesian processing are an example where such uncertainty values are co- or post-computed with the predicted labels, and such Bayesian processing is envisaged herein in embodiments. An example of Bayesian processing in ML is described in A. Kendall et al in "What Uncertainties Do We Need in Bayesian Deep Learning for Computer Vision?", published in NIPS'17: Proceedings of the 31st International Conference on Neural Information Processing Systems, December 2017, pp 5580-5590. Other methods which provide measures for the predictive uncertainty are equally applicable in this context. Here different probabilistic neural network approaches, Bayesian- and non-Bayesian uncertainty estimation schemes as well as stochastic-gradient Monte-Carlo Markov Chain (MCMC) methods or simpler ensemble techniques can be used depending on the envisaged prediction task.

It is then the objective in one embodiment to adjust the acquisition parameters at step S630 so as to decrease, in instances minimize, the uncertainty value. The uncertainty values $\sigma$ associated with the respective predictions $\ell$ are a function (see (3) above) of the frequency $f$. Instead of minimizing for uncertainties, a "dual" formulation is also envisaged herein, wherein the task is to maximize for certainties. Both optimizations are equivalent for present purposes.

Where the frequencies are drawn from a discrete set, the adjustments at step S630 involves selecting the frequency with the lowest uncertainty, or at least with an uncertainty below a defined threshold. If more than one frequencies satisfy this threshold-condition, a random choice may be made, or the user is offered through the user interface UI, a choice to select from among the qualifying frequencies.

If the frequency is a continuous function S of the uncertainty $\sigma$, a gradient of this function may be computed, and the frequency is adjusted at a certain step width along the gradient so as to decrease the uncertainty.

In some embodiments, in addition to basing the prediction on the current image, it is also the current frequency that is used to predict S620 the one or more object properties. This further constraint allows making the prediction yet more robust and/or may allow quicker training of the implementing ML model.

In some embodiments, the adjustment step S630 is not based on a single image, the current image in the stream, but is based on a "stack", or sequence of images, pre-acquired in an exploratory phase and it is this stack of images that is then used in the adjustment step S630. An exemplary embodiment for said stack-based parameter $f$ adjustment S630 is as in the embodiments of Figure 4, but other stack-based embodiments are also envisaged. Stack based processing at S630 yet further improves robustness as this allows to account for spatial and temporal correlation in the imaged object.

The adjustment step at S630 of choosing the best frequency may be based on minimizing the uncertainty-versus-frequency function $S$. The minimization is preferably done on the fly. The frequencies at S630 may be obtained through prior knowledge by look-up in a look-up table or other memory, where each label is pre-associated with a respective best frequency. One embodiment for how this step may be implemented is as described above in Figure 3.

In the proposed method, the frequency can be computed, in parallel or in series, for some or each active source of the transducer separately, per pixel. Alternatively, at a coarser graining, the frequencies are computed per sub-sets of the image. In an extreme case, instead of operating locally, global embodiments are also envisaged where a global label with associated frequency is computed for the whole of the image.

As mentioned earlier, the method of concatenating prediction and adjustment as proposed in the method provides for good robustness. Specifically, the proposed prediction S620, on which the parameter adjustment S630 is based, forms a semantic layer. The semantic layer represents an "abstraction away" from the particular pixel value pattern of a given image that is prone to noise corruption, thus making the proposed label based frequency adaptation more robust.

As mentioned earlier, according to one embodiment a machine learning model M used has a neural-network (NN architecture), in particular a convolutional neural-network (CNN) architecture as shown in Figure 7A, to which reference is now made.

Broadly, the NN structure of the machine learning component includes a plurality of nodes, at least partly interconnected and arranged in different layers. The layers are arranged in one or more sequences. Each node is an entry capable of assuming a value and/or can produce an output based on input it receives from one or more nodes of an earlier layer.

Each node is associated with a certain function which can be a simple scalar value (node weight) but can also be with more complex linear or non-linear functions. A "connection" between nodes in two different layers means that the node in the later layer can receive an input from the node in the earlier layer. If there is no connection defined between two nodes, no output of one of the two nodes can be received by the other node as input. The node produces its output by applying its function to the input. This can be implemented as a multiplication of the received input by the scalar value (the weight) of the node. The interconnected layered nodes with their weights, layer size etc. forms a NN (neural network) model as one embodiment of the ML model envisaged herein. The model may be stored in a matrix or tensor structure in a memory. Once trained, this structure forms the trained machine learning component which can be held on one or more memories SM.

Figure 7A is an exemplary embodiment of a suitable CNN configuration for inferring the object property labels $\ell_k$ from a current input image $I_k$, or from a sequence/stack of such images.

Model M is preferably a deep neural network including one or more hidden layers. The layers L$i$ are hidden layers as there are arranged between an input layer and an output layer. The network M consists of several convolutional filter layers $L_i$, some or each employing one, or a multitude of, convolutional filter kernels. Some or each of the convolutional filter layers are followed by an activation layer and in some embodiments, a pooling layer (not shown). Optionally, there is also one or both of a batch normalization and dropout layer. Further optionally, there are in addition one or several fully connected layers. The above mentioned series of layers terminates in a classification layer producing the prediction of the labels $\ell_k$. In addition, a probability estimate may be provided for each label. In one embodiments this is achieved by including parametrized probability distributions or densities, and these parameters are estimated alongside the weights of the model M, and may then be used to compute, for example, a respective probability for $\ell_k$ that represents the respective uncertainty. In addition or instead, the uncertainty may be represented as a variance or standard deviation. In the Figure 7A, "$\sigma$" is used to indicate the said uncertainty, but this is merely symbolic and is to include any suitable statistical quantity capable of representing the uncertainty such as, as said, a probability or entropy, higher moments, or any other.

The activation layers determine the (typically non-linear) function with which output values from one layer are modified and provided as input to the next layer. The pooling layer combines outputs from multiple elements of one layer to serve as input to a single element in the next layer. Combined, each convolutional, activation and pooling layer serves to process data non-linearly, change its dimensionality, and pass it on to the next layer. The parameters of each of the convolutional layers are "learned" (optimized) in the training phase. The number of parameters in each convolutional layer depends on the size of the convolutional kernel, number of kernels, and the step size "stride" when moving over the image processed at a given layer. The number of parameters for fully connected layers is determined by the number of elements in the previous and current layers.

Optionally, additional input parameters $a_k$ may be incorporated into the model to improve the accuracy and generalization ability of the model. These non-image parameters - usually categorical, sparsely represented data - can be represented by low-dimensional embeddings (e.g. one-hot encoding). For instance, such embeddings can be processed by one or more fully connected layers, and are then point-wisely added or concatenated to an arbitrary intermediate feature map (layer) by tiling the output from the one or more fully connected layer over the spatial dimensions of the feature map. In one embodiment, additional input parameters $a_k$ include the current acquisition parameter $f_k$, such as US frequency, for the current image.

Once trained, during deployment, real data, such as a current image $I_k$ [or $(I_k, f_k)$] is applied to an input layer INL. From the input layer INL, the image data $I_k$ propagates through application of the filters represented by the hidden layers $L_1 - L_N$. The number or layers $L_1 - L_N$ are two, three or much more in the order of tens (e.g., 20-50 or other). The image data $I_k$ is transformed during the propagation to then emerge as a feature vector OUTL. The non-image contextual data $a_k$ may be propagated through a different strand of the model (not shown) including one or more fully connected layers. The outputs of these two strands, the strand $L_1 - L_N$ that processes the image data $I_k$ and the strand that processes the (possibly non-image) contextual data $a_k$ may be merged as will be explained later in more detail below with further reference to embeddings.

The feature vector OUTL could be considered as a low-dimensional ID representation of the input image $I_k$. The feature vector OUTL may then be followed by a single, or a plurality of, task-specific layers (not shown) such as fully connected or convolutional layers with an activation function (e.g. any one or more of linear, sigmoid, softmax, tangent hyperbolic) depending on the task. The task is a regression or a classification, preferably the latter. For classification tasks, these feature vectors are encodings of the classes contained in the input image and can be mapped to class probabilities by appropriate normalization / mapping functions (e.g. softmax etc.). For segmentation or other regression tasks, the abstract feature vector is further processed by additional (network) layers. These task specific layer(s) is/are applied to the (encoded) feature vector layer OUTL to predict e.g. the position/contour and class labels of object properties as detected. The predicted labels are represented as output $\hat{\ell}_k$. The output labels may be represented as a vector, with entries for one type of property, or the classification output is represented as a matrix the size of the input image, each entry representing a label per pixel, eg pixel $(i,j)$ is a plaque pixel, whilst pixel $(i,j+1)$ is a vessel pixel, etc. A smaller matrix feature map may implement a coarser prediction, with labels per regions (subset of pixels). etc. Whilst classification or classification-type tasks are mainly envisaged, regression or regression-type tasks such as predicted measurements in relation to a certain anatomy, eg lumen diameter etc, are also envisaged, instead of, or in addition to, the classification.

One, more than one, or all hidden Layers $L_i$ are convolutional layers and their operation can be defined in terms of the filters. Convolutional layers as used in model M are to be distinguished from fully connected layers. In convolutional layers, each node is connected only to a sub-set of (pooled) nodes of the previous layer. The node in a follow-up layer is computed by forming a filter operation based on a sub selection of nodes from the previous layer. For different nodes of the follow-up layer, different sub-sets of the previous layer are processed, preferably with the same filter. The filter is a collection of numbers $n_{ij}$ arrangeable in a matrix. In some embodiments, the filter operation includes forming sums of products $n_{ij} \cdot m_{jk}$, $m_{jk}$ being a node of the previous layer. This sum-of-product operation is hence akin to traditional convolutional or filter operation where a filter mask is slid over matrix data. In general, the layers are implemented as matrices. The filter operations that implement the propagation of the data through the layers are implemented as matrix operations and are preferably run on GPUs or other processors capable of parallel processing such as those with multi-core designs or others. In fully connected layers, each node of the previous layer is connected to a node in the current layer using a learnable activation weight.

As mentioned above, fully connected layers are also envisaged herein for processing of non-image data, such as bio-characteristics of the patients. The fully connected layers are envisaged to form a separate strand of processing layers (not shown) in which one, two or more of such fully connected layers are arranged in sequence and through which the non-image data , is passed. Optionally, this data is also passed through a convolutional layer, separate and different from the above described convolutional layers for processing of the image. The output of the last of those fully connected layers for non-imaging data can be concatenated to one of the hidden layers in the CNN branch of the model thereby merging non-image data with image data. In this way the output, that is the labels, not only takes into account the spatial data in the image data but also the contextual data such as the current acquisition parameter.

As mentioned, embeddings such as one-hot-encodings, can be used to represent categorical sparse data as vectors. The non-image data processing strands may include a combination of fully connected and convolutional layers. Also, the image-data processing strand as shown in Figure 7A may include sequences of one or more fully connected layers, in particular when non-image data $a_k$, is jointly processed and passed through the layers as augmented data $(I_k, a_k)$ In addition to convolutional layers and fully connected layers, either one or both strands may further include at any one or more of pooling layers, activation layers drop-out layers, in any combination. In addition, there may be deconvolutions layers that may be thought to represent an approximation of a reverse operation to the convolutional layers. The number of nodes produced at each layer may change in dimension, so size (number of rows and columns) and/or depth an output layer may grow or shrink. The dimension of the output of a convolutional layer is in general the same as the dimension of the input, or less, depending on its "stride" which represents how many nodes the filter is effectively slid past for each operation. Thus a down-sampling may occur. An up-sampling is also envisaged with deconvolutions layers. Some architectures include one or more runs of both, up-sampling layers followed by down-sampling or the other way around. In general, the architecture of Figure 7A is a net down-sampling as the network acts as a mapping that maps the input image $I_k$ down to labels $\hat{\ell}_k$, if this is vector.

Referring now to the training phase or process, this is implementable by a training system TS shown in Figure 7B. The training is based on suitable training data. Training data can be obtained by acquiring real imagery or by obtaining simulated US-images, both denoted herein as $I_k$. Real US-imagery may be retrieved as historical image data from patient records such as PACS or from other image repositories.

In more detail, the training involves acquisition of real or simulated X-ray images $I_k$ ($k \in 1...K$), paired with "ground truth" labels $\ell_k$ that encode properties in relation to the image one or more objects such as anatomies, etc.

The pairs $\{I_k, \ell_k\}$ are used in the training process, in which the images $I_k$ are provided as input to a convolutional neural network (CNN), and the network parameters are optimized to infer the prediction vector $p_k$ as output. Optionally, the images can be paired with additional patient-specific parameter vectors (such as height, weight, age, gender, ethnicity, BMI, anatomical abnormalities, implanted devices, etc.) as input. Although one suitable CNN configuration for the present object property label prediction is the one shown in Figure 7A other architectures are also envisaged herein, and so are models other than NN such as support vector machines (SVM), decision trees, random forest and others still.

With continued and more detailed reference to Figure 7B, the computerized training system TS as may be used for training the machine learning model M based on training data $(X, Y)^j$. "$X$" represents suitable training imagery $I_k$, whilst "$Y$" represents the associated label $\ell$.

The training data pair can be obtained in a manual labelling exercise by a human expert. Alternatively, and preferably, the labeling is retrieved in an automated manner. For example, the labels associated with given training image may be assembled from metadata such as DICOM header data or may be retrieved from patient records as held in databases. A suitably programmed scripting tool may be used to accomplish finding the associated object property/properties label(s) $Y=\ell_k$ for each image training $X=I_k$.

The training data is thus organized in pairs $j$ in particular for supervised learning schemes as mainly envisaged herein. However, it should be noted that non-supervised learning schemes are not excluded herein.

The machine learning model M, such as the shown CNN network in Figure 4A or a hybrid network comprising CNN strands for processing image data $I_k$ and fully connected layer strands for processing of non-image data $a_k$ such as associated acquisition parameter $f$ is chosen, and the weights are pre-populated with some initial, possibly random or uniform values. For instance, the weights for all nodes may all be populated by "$l$"s or other numbers.

The weights $\theta$ of the model M represent a parameterization $M^\theta$, and it is the object of the training system TS to optimize and hence adapt the parameters $\theta$ based on the training data $(X^j, Y^j)$ pairs. In other words, the learning can be formalized mathematically as an optimization scheme where a cost function is minimized although the dual formulation of maximizing the utility function $F$ may be used instead.

Assuming for now the paradigm of a cost function $F$, this measures the aggregated residue(s), that is, the error incurred between data estimated by the model M and the targets as per some or all of the training data pairs $j$:

$$argmin_\theta F = \sum_j || M^\theta(X^j) - Y^j || \qquad (4)$$

More specifically, the image data $X$ of a training pair is propagated through the initialized network M. X for a first pair "$j$" is received at an input IN, passed through the model and then received at output OUT as output training data $M^\theta(X)$. A Suitable measure $|| \cdot ||$ is used such as a $p$-norm, squared differences, or other, to measure the difference between the actual output $M^\theta(X)$ and the desired output Y.

The output training data $M(X)$ is an estimate for the label $\ell$ given the input training image data X. In general, there is an error between this output $M(X)$ and the associated target $Y$ of the presently considered pair. An optimization scheme such as backward/forward propagation or other gradient based methods may then be used to adapt the parameters $\theta$ of the model $M^\theta$ so as to decrease the residue for the considered pair $(X, Y)^j$ or a subset of training pairs from the full training data set.

After one or more iterations in a first, inner, loop in which the parameters $\theta$ of the model are updated based on the optimization scheme used, the training system TS enters a second, an outer, loop where a next training data pair $X^{j+1}, Y^{j+1}$ is processed accordingly. This inner loop may be implemented by one or more forward and backward passes in the forward/backpropagation algorithm. However, this time while adapting the residue, it is not only the individual residue for the current pair that is adapted but the aggregated, summed, residues of all the training pairs considered up to this point in time are adapted if required to improve the objective function. The aggregated residue can be formed by configuring the objective function *f* as a squared summed (or other algebraic combination) such as in eq (4) of some or all considered residues for each pair.

The generalized training system as shown in Figure 7B can be considered for all learning schemes, in particular supervised schemes. Unsupervised learning schemes that perform suitable clusterings in phase space, augmented or not, may also be envisaged. GPUs may be used to implement the training system.

Referring now the flow chart in Figure 8, this shows a computerized method for training a machine learning model MLC, in particular as explained above in Figures 4A, B.

At step S810 training data is received in the form of pairs including US or OCT imagery and associated labels that identify ROIs, findings, or other properties of one or more objects. For present purposes, the label may in addition or instead code for an imaging purpose or task given the image, the said task or purpose being thus a "property" of a particular object, such as anatomy.

At step S820, the imagery, and optionally an associated acquisition parameter *f* or other non-image contextual data, is applied to an initialized machine learning model M to produce an output.

A deviation of the training output from the associated label is quantified by a cost function *F*. One or more parameters of the model are adapted at step S830 in one or more iterations in an inner loop to improve the cost function. For instance, residues as measured by the cost function are decreased when adapting the model parameters.

The training method then returns to step S810 where the next pair of training data is fed in to enter an outer loop. In step S820, the aggregated residues of all pairs considered up to that point are decreased, in particular minimized.

More generally, the parameters of the model M are adjusted to improve an objective function *F* which is either a cost function or a utility function.

The components of the image processor PR may be implemented as one or more software modules, run on one or more general-purpose processing units TS such as a workstation associated with the imager IS, or on a server computer associated with a group of imagers.

Alternatively, some or all components of the image processor PR, in particular the predictor component PA and the parameter adjuster PA, may be arranged in hardware such as a suitably programmed microcontroller or microprocessor, such an FPGA (field-programmable-gate-array) or as a hardwired IC chip, an application specific integrated circuitry (ASIC), integrated into the imaging system IS. In a further embodiment still, the image processor PR may be implemented in both, partly in software and partly in hardware.

The different components of the image processor PR may be implemented on a single data processing unit. Alternatively, some or more components are implemented on different processing units, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems. Transitory storage media are also envisaged in embodiments.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims, be they numerals, alphanumerical, or a combination of one or more letters, or a combination of any of the foregoing, should not be construed as limiting the scope.

**Claims**

1.   An imaging system (IS), comprising:

an image acquisition unit (AQ) for acquisition of image data (11) of at least a part of an object (OB), the said acquisition being based on an imaging signal imitable by the unit (AQ) to interact with the object, the image acquisition unit (AQ) adjustable to operate at different acquisition parameters that determine at least in part a property of the imaging signal;
a predictor component (PC) configured to predict, based at least on the acquired image data (11), one or more properties of the object; and
an acquisition parameter adjuster (PA) configured to adjust, based on the predicted one or more properties, the acquisition parameter at which the image acquisition unit (AQ) is to acquire follow-up image data (12).

2.   System of claim 1, wherein the acquisition parameter is adjusted automatically by the acquisition parameter adjuster (PA) so as to increase image quality.

3.   System of claim 1 or 2, wherein the predictor component (PC) is to predict plural object properties associated with a respective uncertainty value, wherein the acquisition parameter adjuster (PA) is to adjust the acquisition parameter based on the uncertainty value or on a gradient thereof.

4.   System of claim 3, wherein the acquisition parameter adjuster (PA) is to adjust the acquisition parameter so as to decrease the uncertainty value of the predicted one or more properties.

5.   System of any one of the previous claims, wherein the predictor component (PC) is to predict the one or more object properties based on a current acquisition parameter.

6.   System of claims 3 to 5, wherein the uncertainty value is provided by a user via a user interface (UI).

7.   System of any one of the previous claims, wherein the predictor component (PC) includes a pre-trained machine learning model.

8.   System of claim 7, wherein the pre-trained machine learning model includes a neural network.

9.   System of any one of the previous claims, wherein the image acquisition unit (AQ) includes a multi-frequency ultrasound imaging device, wherein the imaging signal is an ultrasound signal.

**10.** System of claim 9, wherein the acquisition parameter includes a frequency of the ultrasound signal.

**11.** System of claim 9, wherein the ultrasound imaging device is an intravascular ultrasound, IVUS, imaging device.

**12.** An imaging method, comprising the steps of:

acquiring (S610) image data (11) of at least a part of an object (OB), the said acquiring being based on an imaging signal imitable by an image acquisition unit (AQ) to interact with the object, the image acquisition unit (AQ) adjustable to operate at different acquisition parameters that determine at least in part a property of the imaging signal;
predicting (S620), based at least on the acquired image data (11), one or more properties of the object; and
adjusting (S630), based on the predicted one or more properties, the acquisition parameter at which the image acquisition unit (AQ) is to acquire follow-up image data (12).

**13.** A training method configured to train the predictor component (PC) as per claim 7.

**14.** A computer program element, which, when being executed by at least one processing unit (PR,TS), is adapted to cause the at least one processing unit (PR,TS) to perform the method as per claim 12 or 13.

**15.** A computer readable medium having stored thereon the program element of claim 14.

**FIG. 1**

**FIG. 2**

**FIG. 3**

$$f_S = \text{argmin } \sigma(f_i)$$
$$f_i$$

FC

PC

$\ell_{1,\ldots,N}$

PA

$t = 0$

$f$

$I_{f_1,\ldots,fN}$

$\sigma_{1,\ldots,N}$

MUX

$\ell_S = \ell^{(fs)}$

PC

$p_S$

PA

$t = 1$

$f_S$

$I_{fs}$

$\sigma_S$

$\sigma > T$

Y/N

MUX

$\ell_S$

$$f_S = \text{argmin } \sigma(f_i)$$
$$f_i$$

**FIG. 4**

**FIG. 5**

I , f

S610

S620

S630

S640

FIG. 6

**FIG. 7A**

**FIG. 7B**

FIG. 8

Europäisches
Patentamt

European
Patent Office

Office européen
des brevets

## EUROPEAN SEARCH REPORT

Application Number

EP 20 16 7460

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/020770 A1 (KONINKLIJKE PHILIPS NV [NL]) 30 January 2020 (2020-01-30) * paragraphs [0001], [0022], [0023], [0028], [0030], [0033], [0034], [0042], [0060] * * figures 1,2 * | 1-15 | INV. A61B8/00 G01S15/89 G01S7/52 G01S7/539 G06K9/32 |
| X | US 8 784 318 B1 (NAPOLITANO DAVID J [US] ET AL) 22 July 2014 (2014-07-22) * column 5, line 44 - column 6, line 27 * * figure 3 * | 1 | |
| X | WO 2018/130370 A1 (CONTEXTVISION AB [SE]) 19 July 2018 (2018-07-19) * page 11, line 24 - page 12, line 26 * * figure 3 * | 1 | |

TECHNICAL FIELDS
SEARCHED     (IPC)

A61B
G01S
G06K

The present search report has been drawn up for all claims

| Place of search | Date of completion of the search | Examiner |
|---|---|---|
| Munich | 5 August 2020 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 7460

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2020

| Patent document cited in search report | | Publication date | Patent family member(s) | | Publication date |
|---|---|---|---|---|---|
| WO 2020020770 | A1 | 30-01-2020 | NONE | | |
| US 8784318 | B1 | 22-07-2014 | US 8784318 B1 | | 22-07-2014 |
| | | | US 2015073276 A1 | | 12-03-2015 |
| WO 2018130370 | A1 | 19-07-2018 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2

- **A. KENDALL et al.** What Uncertainties Do We Need in Bayesian Deep Learning for Computer Vision?. *NIPS'17: Proceedings of the 31st International Conference on Neural Information Processing Systems,* December 2017, 5580-5590